# EUROPEAN PATENT APPLICATION

(11) **EP 3 657 171 A1**
(43) Date of publication of application: **27.05.2020**
(21) Application number: 18207281.9
(22) Date of filing: 20.11.2018
(51) Int. Cl.: G01N 33/574, G16H 50/20

(54) **METHOD FOR THE DETERMINATION OF THE PROGNOSIS OF OVARIAN CARCINOMA (OC)**

(71) Applicant: Philipps-Universität Marburg, 35037 Marburg (DE)
(72) Inventor: MÜLLER, Rolf, 35041 Marburg (DE); WAGNER, Uwe, 35274 Schönbach (DE)
(74) Representative: Stumpf, Peter

(57) **Abstract**

The invention describes a method for determining and predicting the prognosis of an individual patient with diagnosed ovarian carcinoma (OC). The method comprises the steps measuring the concentration of the markers HSPA1A, BCAM, and DKK1 and the markers CAPG, LCK and TNFAIP6 in the biological sample of said individual, calculating multi-marker scores for the biological sample of the said individual, and indicating a short-term survival or a long-term survival for the said individual using the multi-marker scores.

## Description

### Technical Field of the Invention

This invention concerns a method for the determination of the prognosis of an ovarian carcinoma in individual patients. OC is the common abbreviation of ovarian carcinoma. The determination of the prognosis allows a prediction of the survival time, especially a distinction of short-term and long-term relapse-free survival time. The prognosis depends on whether OC growth recurs and, if so, on the time to relapse. This means that the prognosis for an individual depends on the probability of recurrence and the expected time to relapse. If the probability of recurrence is low and/or the expected time to relapse is long, the prognosis is good. If the probability of recurrence is high and/or the expected time to relapse is short, the prognosis is bad.

OC is a malignant tumour of the ovaries or fallopian tubes (oviducts) in humans or animals, and occurs as solid tumour masses and peritoneal carcinomatosis. OC and OC precursor lesions comprises 2C73.0, 2C74, 2D90, 2D91, and XH8D74 according to ICD-11 classification. OC is usually diagnosed at an advanced stage, since suitable methods for early detection are not available. OC is the deadliest of all gynaecological malignancies and shows an overall 5-year survival rate of less than 50%. The most common form of OC is high-grade serous ovarian cancer (HGSOC). HGSOC makes up the majority of OC cases and has the lowest survival rate.

The peritoneal fluid is the free fluid in the abdominal and pelvic cavities (peritoneal cavity). Many diseases are accompanied by the production of large volumes of peritoneal fluid, which is then referred to as ascites. Some scientists however use the term "ascites" as a synonym for peritoneal fluid. Sampling of peritoneal fluid is generally performed by paracentesis.

The causes of ascites can be manifold. One possible cause of ascites is the growth of a tumour in the abdominal or pelvic cavities. In patients with OC, tumour cells can be shed at a very early stage of the disease and spread within the abdominal cavity via the ascites to form metastases on the peritoneal wall, in the omentum, and the serous membranes of other organs. Even though first-line therapy (surgery and chemotherapy) frequently achieves a clinically tumour-free state, most patients relapse due to the outgrowth of residual cancer cells.

Markers (biomarkers) are substrates of the human or animal body or in human or animal body fluids such as blood, serum, plasma, or peritoneal fluid (ascites), which are useful for diagnosis of diseases and/or for determination of the prognosis of a disease. Markers are measurable indicators of the presence or severity of a disease. The concentration of markers is measured (determined) in order to obtain a diagnosis and/or prognosis. The concentration of markers can be increased or decreased, depending on the marker and the disease. Markers can be proteins, peptides, or oligopeptides; they also can be low molecular mass organic substances or elements.

The relapse-free survival time (RFS) is the time between the first-line therapy and the relapse of the disease. Long-term survivors are patients who survive OC for at least five years after the beginning of the first-line therapy. Long-term relapse-free survivors are patients with no relapse at 24 months, which means that the censored or uncensored relapse-free survival time is ≥24 months. Short-term relapse-free survivors are patients with relapsed OC within 24 months after first-line therapy, which means that the uncensored relapse-free survival time is <24 months. The individual prognosis (probability of recurrence and expected time to relapse) is of great clinical relevance, because it is a key element of a patient's quality of life, for patient counselling and the choice of appropriate individual therapy. OC usually has a poor prognosis, because of the lack of any early detection or screening test, so that most cases are diagnosed at an advanced stage.

OC metastasizes at an early stage, usually before it has been diagnosed. Typically, OC metastasizes within the peritoneal (abdominal and pelvic) cavity by transcoelomic dissemination. Although OC is the deadliest cancer of the female reproductive tract with an average relapse-free survival time after first-line therapy of less than 2 years, a small fraction of patients (<20%) remains relapse-free for more than 5 years, and some even considerably longer. To date, biomarkers that can reliably distinguish these patients are not available. Neither single markers in tumour tissue or in the malignancy-associated peritoneal fluid due to ascites nor transcriptomic analyses have succeeded in defining clinically applicable markers or signatures.

### State of the Art

The following methods are used for the determination of the prognosis of OC in individual patients:
- Statistical analysis: Statistical analysis of the survival rates of OC can be quite accurate because large evaluable populations are available. For example, the relative five-year survival for patients with stage IA OC is approximately 92% and for patients with stage IV OC is approximately 20%. However, this statistical information does not apply to the prognosis for an individual patient, because the statistical evaluation of a population is not meaningful for the prognosis of an individual.

Additionally, different subtypes of OC show very different five-year survival rates. For example, endometrioid OC has a five-year survival rate of 82%, whereas high-grade serous adenocarcinoma has a five-year survival rate of approximately 44%. - Determination of prognostic factors: A number of prognostic factors has been proposed for the determination of the prognosis of OC. There are positive prognostic factors, which indicate better chances of survival and negative prognostic factors, which indicate worse chances of survival.

Positive, yet unreliable prognostic factors are for example no residual disease after surgery (complete initial tumour debulking), high primary platinum sensitivity, infiltration by cytotoxic CD8+ T cells, BRCA2 mutations, age under 45 years, non-serous type of OC, low histologic grade, early stage, co-occurrence with endometrial cancer, and low CA125 levels.

Negative, yet unreliable prognostic factors are for example rupture of the ovarian capsule during surgery, incomplete initial tumour debulking, low primary platinum sensitivity, age over 45 years, high-grade serous type of OC, stage IV OC, high histologic grade, clear cell type OC, upper abdominal involvement, high CA125 levels, presence of tumour cells in the blood, and high levels of IL-6, IL-10, and/or TGF-beta in peritoneal fluid due to ascites.

Gene expression can also be a prognostic factor for OC. For example, transcriptional signatures indicative of the immune-reactive subtype are associated with a longer survival, while signatures indicative of the mesenchymal subtype are associated with a short survival. In addition, the expression of numerous genes and proteins in tumour tissue has been associated with either an improved survival (e.g. estrogen receptor) or a poor prognosis (e.g. let-7b, HIF1A, EPHA1, WT1). There are also prognostic factors with conflicting evidence, for example BRCA1 mutation. All of these prognostic factors indicate at best statistical survival probabilities for cohorts of OC patients. They do not predict the survival time or time-to-relapse for an individual patient.

A number of markers in the blood and/or peritoneal fluid due to ascites have already been analysed in a non-systematic manner and suggested for the determination of the prognosis of OC, but none of them was clinically validated as a suitable prognostic marker.

These markers, like many other biomarkers, are proteins, peptides, or oligopeptides. Their concentration in a biological sample can be measured by different methods. One method for example employs the SOMAscan® Proteomic Assay by SomaLogic Inc. The SOMAscan® Proteomic Assay currently measures up to 1,300 markers or protein analytes in a biological sample for instance in serum, plasma, peritoneal fluid, or cerebrospinal fluid. The SOMAscan® Proteomic Assay is able to quantify markers that span over eight logs in abundance from femtomolar to micromolar. The SOMAscan® Proteomic Assay uses aptamer technology. The employed aptamers (SOMAmer reagents, SOMAmers) have a dual nature as protein affinity-binding reagents with defined three-dimensional structures, and unique nucleotide sequences recognizable by specific DNA hybridization probes. The SOMAscan® Proteomic Assay transforms the protein present in a biological sample into a specific DNA signal. The results of standard DNA quantification techniques are normalized to assure data consistency by correcting for variations due to the SOMAscan® Proteomic Assay, thereby allowing for comparison of samples across a plate. The normalized results obtained for replicate calibration samples included in the assay are then compared to a previous established reference calibration value. This generates a calibration scale factor that is applied to all samples. The final data are reported in relative fluorescent units (RFU, unit) after normalization and calibration. A strong advantage of the SOMAscan® Proteomic Assay over mass spectrometry is its applicability to unfractionated blood plasma and related fluids like as peritoneal fluid due to ascites in spite of the high abundance of blood proteins such as albumin, globulins, and fibrinogen, which has been a limiting factor for all previous mass spectrometry-based proteomic analyses of peritoneal fluid.

Therefore, there are currently no suitable methods for the determination of the prognosis of OC in an individual. Neither single markers in tumour tissue or in the malignancy-associated peritoneal fluid due to ascites nor transcriptomic analyses have succeeded in defining clinically applicable markers for the prediction of the prognosis in an individual. A serious limitation of all previous efforts is the lack of any unbiased global analyses for the discovery of OC prognostic markers, an approach used for the present invention.

### Problem

There is currently no reliable method for the determination of the prognosis of OC in an individual.

This invention solves therefore the following task:
- Improved determination of the relapse-free survival time (relapse-free survival, RFS) of individual patients with OC as a basis for improved therapy.

### Solution

Claim 1 solves the inventive task. The main inventive steps are first the identification of the most significant markers in a biological sample (e.g. peritoneal fluid) that have the highest significance to the prognosis in diagnosed OC of an individual and second the biostatistical delineation of combinations of these markers that allow for a clear distinction of short-term and long-term relapse-free survivors. This invention describes the suitable markers for the determination of the prognosis of OC in an individual and the biostatistical analysis of these markers.

The key feature of the invention is the selection of markers from a very large number of proteins, peptides, and/or oligopeptides in a biological sample such as peritoneal fluid determined by an unbiased analysis that enables a reliable determination of the prognosis of OC. The inventors identified several markers out of 1,305 potential markers, which allow for an improved prediction of prognosis in patients with OC. In order to perform the inventive method a biological sample such as peritoneal fluid of an individual must be provided. Other biological samples may be blood, plasma, serum or other non-solid biological tissues. These samples are obtained from patients with diagnosed OC. Usually these samples are provided by a physician. The most useful biological sample to apply the invention is peritoneal fluid. Sampling of peritoneal fluid is generally performed by paracentesis.

The inventive method for the determination of the prognosis of an ovarian carcinoma (OC) in a biological sample from an individual with diagnosed OC, comprises the steps
(i) measuring the concentration of at least the markers HSPA1A, BCAM, and DKK1 (markers identifying all long-term relapse-free survivors) in the biological sample of said individual;
(ii) measuring the concentration of at least the markers CAPG, LCK, and TNFAIP6 (markers identifying all short-term relapse-free survivors) in the biological sample of said individual;
(iii) determining for each marker from step (i) whether the concentration of the marker is above or below the threshold that separates short-term relapse-free survivors and long-term relapse-free survivors;
(iv) determining for each marker from step (ii) whether the concentration of the marker is above or below the threshold that separates short-term relapse-free survivors and long-term relapse-free survivors;
(v) assigning a score of 1 for all markers of step (i) with a concentration below the threshold and a score of 0 for all markers of step (i) with a concentration higher than the threshold;
(vi) assigning a score of 1 for all markers of step (ii) with a concentration below the threshold and a score of 0 for all markers of step (ii) with a concentration higher than the threshold;
(vii) addition of the scores of step (v);
(viii) addition of the scores of step (vi);
(ix) division of the sum of step (vii) by the number of the markers of step (i) in order to yield a signature score 1;
(x) division of the sum of step (viii) by the number of the markers of step (ii) in order to yield a signature score 2;
(xi) determination whether both signature scores are consistent so that both are below or above 0.5;
(xii) calculating a multi-marker score in case of consistent signatures from step (ix) and (x) by addition of the scores from step (vii) and from step (viii) followed by division by the total number of markers (number of markers in signature 1 plus number of markers in signature 2);
indicating a short-term relapse-free survival in said individual if the multi-marker score of step (xii) is 0.5 or higher, and indicating a long-term relapse-free survival in said individual if the multi-marker score of step (xii) is below 0.5.

The thresholds for each marker according to step (iii) and (iv) which concentration is measured by SOMAscan® Proteomic Assay can be seen in Table 1, third column (best-fit threshold (Units)). If the concentration of the markers is measured by another method, like as ELISA, other antibody-based techniques, microarray-based technology, other target-proteomics-based methods or mass spectrometry the thresholds for each marker must been adjusted.

The first step of the inventive method is the measuring (determining) of the concentration of the markers HSPA1A, BCAM, and DKK1 in a biological sample obtained from an individual with diagnosed OC. These markers are used to identify all long-term relapse-free survivors. In another embodiment, the concentration of the markers HSPA1A, BCAM, DKK1, Corticotropin-lipotropin, and DPP7 is measured in step (i). In another embodiment the concentration of the markers HSPA1A, BCAM, DKK1, Corticotropin-lipotropin, DPP7, LMAN2, and CTSS is measured in step (i). In another embodiment the concentration of the markers HSPA1A, BCAM, DKK1, Corticotropin-lipotropin, DPP7, LMAN2, CTSS, RPSA, and ARSB is measured in step (i).

The next step of the inventive method is the measuring (determining) of the concentration of the markers CAPG, LCK, and TNFAIP6 in a biological sample obtained from a subject with diagnosed OC. These markers are used to identify all short-term relapse-free survivors. In another embodiment, the concentration of the markers CAPG, LCK, TNFAIP6, and REG1A is measured in step (ii). In another embodiment, the concentration of the markers CAPG, LCK, TNFAIP6, REG1A, CTSZ, ARSA, RPS7, CD27, and CRLF1 is measured in step (ii).

These markers are constituents of two kinds of prognostic signatures. The markers HSPA1A, BCAM, DKK1, Corticotropin-lipotropin, DPP7, LMAN2, CTSS, RPSA, and ARSB are constituents of the type 1 signature (type 1 markers). The markers CAPG, LCK, TNFAIP6, REG1A, CTSZ, ARSA, RPS7, CD27, and CRLF1 are constituents of the type 2 signature (type 2 markers). Table 4 shows a description of the abbreviations of the markers.

In order to measure the concentration of these markers in a biological sample any suitable method can be applied, e.g. ELISA-based techniques, mass spectrometry, antibody- or aptamer-based microarrays or any other suitable quantification method, however preferably a SOMAscan® Proteomic Assay. According to the method of measuring the concentrations the thresholds of each marker must been adjusted. In the next step the concentration of each type 1 marker (step i) and of each type 2 marker (step ii) is compared with a threshold, which was defined by the concentration of said markers in patients with known long-term survival (long-term relapse-free survivors) and known short-term survival (short-term relapse-free survivors). The thresholds for the markers which concentration is measured by SOMAscan® Proteomic Assay can be seen in Table 1, third column (best-fit threshold (Units)).

In a next step, the two kinds of prognostic signatures are calculated: Type 1 signatures are composed of at least HSPA1A, BCAM, and DKK1 and identify all long-term relapse-free survivors, yet display a limited specificity (false positives). In an embodiment, the type 1 signatures are composed of at least HSPA1A, BCAM, DKK1, Corticotropin-lipotropin, DPP7, LMAN2, CTSS, RPSA, and ARSB. Type 2 signatures are composed of at least CAPG, LCK, and TNFAIP6 and identify all short-term relapse-free survivors, yet display a limited specificity (false positives). In an embodiment, the type 2 signatures are composed of at least CAPG, LCK, TNFAIP6, REG1A, CTSZ, ARSA, RPS7, CD27, and CRLF1. Prognosis therefore requires the use of both signatures to achieve a high specificity (nearly 100%). Table 2 lists suitable signature combinations. It is important to determine whether the measured marker concentrations (see step (i) and (ii)) are above or below a threshold according to step (iii) and (iv) of claim 1 (see Table 1, column best-fit threshold (Units)) that separates long-term and short-term relapse-free survivors. The calculation of type 1 signature and type 2 signature scores is performed separately as follows: Assignment of a score of 1 for all markers with a concentration below the threshold, otherwise a score of zero, then addition of these scores and division by the number of markers in the respective signature to yield signature score 1 and signature score 2. The maximum score is 1.0.

If both signature score 1 and signature score 2 are consistent (i.e., both below or both above 0.5), prognosis is possible, otherwise the clinical outcome is not predictable. In case of consistent signature scores a multi-marker score is calculated.

The multi-marker score is calculated as the fraction of the combined markers from both signatures that are above the threshold for each marker (see Table 1). If the multi-marker score is below 0.5, the patient is predicted as a long-term relapse-free survivor. If the multi-marker score is 0.5 or higher, the patient is predicted as a short-term relapse-free survivor.

Table 1 shows all markers, which are significantly associated with relapse-free survival.

**Table 1: Patients were split at the quantile (best-fit quantile) yielding the most significant difference in relapse-free survival for each marker. The corresponding threshold concentrations are listed as best-fit threshold (Units). These threshold concentrations are used as threshold in the steps (iii) and (iv) of claim 1 and 5. Significance was determined by logrank test; p-values <0.05 are significant. HR values >1 indicate an association with a short relapse-free survival; HR values <1 indicate an association with a longer relapse-free survival.**

| **Marker** | **best-fit quantile** | **best-fit threshold (Units)** | **p-value** | **Hazard ratio (HR)** |
|---|---|---|---|---|
| HSPA1A | 0.6 | 67940 | 7.8E-06 | 3.95 |
| BCAM | 0.7 | 10006 | 2.6E-05 | 3.67 |
| DKK1 | 0.5 | 5296 | 2.6E-04 | 3.23 |
| TNFAIP6 | 0.4 | 1615 | 3.2E-04 | 3.34 |
| LCK | 0.6 | 433 | 4.3E-04 | 0.29 |
| MYBPC1 | 0.4 | 662 | 4.9E-04 | 0.34 |
| LAMA1 | 0.5 | 16826 | 5.3E-04 | 3.10 |
| PRSS22 | 0.4 | 22938 | 6.2E-04 | 3.04 |
| CTSZ | 0.5 | 9422 | 6.2E-04 | 2.89 |
| MAPK14 | 0.4 | 1234 | 1.1 E-03 | 0.38 |
| MMP16 | 0.5 | 1212 | 1.2E-03 | 0.36 |
| IL1A | 0.4 | 554 | 1.3E-03 | 0.36 |
| GSTP1 | 0.7 | 40261 | 1.3E-03 | 2.84 |
| RET | 0.7 | 915 | 1.4E-03 | 2.71 |
| CAPG | 0.3 | 16436 | 1.5E-03 | 3.72 |
| IL36A | 0.4 | 924 | 1.5E-03 | 0.38 |
| CHRDL1 | 0.3 | 3277 | 1.6E-03 | 0.38 |
| CA4 | 0.5 | 1396 | 1.8E-03 | 2.81 |
| TAGLN2 | 0.7 | 14760 | 2.1 E-03 | 2.77 |
| STK17B | 0.5 | 4261 | 2.3E-03 | 0.38 |
| CCL13 | 0.5 | 698 | 2.4E-03 | 0.37 |
| CAMK2D | 0.6 | 5366 | 2.4E-03 | 0.34 |
| CAMK2B | 0.6 | 2272 | 2.4E-03 | 0.34 |
| SEMA5A | 0.3 | 38585 | 2.4E-03 | 3.28 |
| PLXNB2 | 0.3 | 5895 | 2.4E-03 | 3.10 |
| CLIC1 | 0.6 | 1153 | 2.5E-03 | 0.35 |
| STAT6 | 0.5 | 830 | 2.5E-03 | 0.39 |
| NOV | 0.3 | 1317 | 2.5E-03 | 3.27 |
| CSK | 0.6 | 1845 | 2.6E-03 | 0.35 |
| Fibrinogen | 0.6 | 99081 | 2.7E-03 | 2.42 |
| FGG | 0.3 | 19236 | 2.9E-03 | 2.99 |
| CNTN2 | 0.5 | 893 | 3.0E-03 | 2.58 |
| CAT | 0.4 | 4377 | 3.2E-03 | 0.40 |
| SERPINE2 | 0.4 | 4343 | 3.4E-03 | 0.40 |
| IL18R1 | 0.7 | 10928 | 3.5E-03 | 2.58 |
| RAC3 | 0.3 | 4136 | 3.6E-03 | 0.39 |
| NAGK | 0.6 | 3472 | 3.6E-03 | 0.36 |
| KLK3 | 0.5 | 529 | 3.6E-03 | 0.40 |
| FN1.4 | 0.4 | 135422 | 3.6E-03 | 2.66 |
| PLCG1 | 0.6 | 467 | 3.7E-03 | 0.37 |
| CLEC1B | 0.6 | 1961 | 4.1E-03 | 2.35 |
| STAT3 | 0.6 | 2238 | 4.4E-03 | 0.38 |
| FAS | 0.4 | 3770 | 4.4E-03 | 2.60 |
| CAMK2A | 0.6 | 1135 | 4.4E-03 | 0.38 |
| FN1.3 | 0.3 | 6522 | 4.6E-03 | 2.80 |
| DSG1 | 0.3 | 2148 | 4.6E-03 | 3.26 |
| CA13 | 0.7 | 2881 | 4.7E-03 | 0.32 |
| WNK3 | 0.6 | 629 | 4.8E-03 | 0.38 |
| REG1A | 0.3 | 739 | 4.8E-03 | 0.42 |
| CPB2 | 0.5 | 3889 | 5.0E-03 | 0.42 |
| FSTL1 | 0.6 | 146503 | 5.1 E-03 | 2.32 |
| STX1A | 0.6 | 499 | 5.2E-03 | 0.38 |
| RAC1 | 0.5 | 11922 | 5.2E-03 | 0.42 |
| LYN | 0.5 | 1857 | 5.4E-03 | 0.42 |
| DAPK2 | 0.5 | 2639 | 5.5E-03 | 0.42 |
| HBA1 | 0.6 | 328 | 5.6E-03 | 0.39 |
| CA3 | 0.4 | 1408 | 5.6E-03 | 0.42 |
| SPHK2 | 0.7 | 439 | 5.9E-03 | 2.39 |
| CMA1 | 0.6 | 4181 | 5.9E-03 | 0.39 |
| CST5 | 0.3 | 9961 | 6.0E-03 | 2.72 |
| MAPKAPK3 | 0.3 | 391 | 6.0E-03 | 0.42 |
| EIF5 | 0.5 | 3875 | 6.0E-03 | 2.31 |
| SIGLEC7 | 0.4 | 2478 | 6.1 E-03 | 2.45 |
| MAPKAPK2 | 0.4 | 1769 | 6.2E-03 | 0.43 |
| STIP1 | 0.5 | 6332 | 6.5E-03 | 0.42 |
| IL37 | 0.6 | 1285 | 6.7E-03 | 2.31 |
| APOM | 0.5 | 18753 | 6.8E-03 | 2.33 |
| CRLF2 | 0.3 | 2076 | 7.0E-03 | 0.42 |
| FGF12 | 0.6 | 340 | 7.0E-03 | 0.39 |
| CTB-50L17.10 | 0.3 | 2024 | 7.2E-03 | 2.76 |
| CASP3 | 0.5 | 15468 | 7.2E-03 | 0.43 |
| CCDC80 | 0.6 | 75629 | 7.4E-03 | 2.31 |
| RASA1 | 0.7 | 882 | 7.6E-03 | 2.44 |
| SERPINA1 | 0.4 | 2182 | 7.8E-03 | 2.40 |
| NAPA | 0.6 | 2701 | 7.9E-03 | 0.41 |
| CD27 | 0.4 | 1078 | 8.1 E-03 | 0.45 |
| MAP2K1 | 0.4 | 1155 | 8.3E-03 | 0.45 |
| LTBR | 0.7 | 5034 | 8.3E-03 | 2.31 |
| DCTN2 | 0.5 | 1364 | 8.4E-03 | 0.43 |
| TGFB3 | 0.5 | 1018 | 8.5E-03 | 2.28 |
| ACP1 | 0.7 | 4182 | 8.5E-03 | 0.37 |
| CFH | 0.7 | 37566 | 8.6E-03 | 2.27 |
| SMAD2 | 0.6 | 3702 | 8.8E-03 | 0.41 |
| PDGFRA | 0.3 | 12742 | 8.9E-03 | 2.66 |
| TNFRSF11 B | 0.6 | 7846 | 9.0E-03 | 2.27 |
| RNASEH1 | 0.5 | 315 | 9.4E-03 | 0.44 |
| VAV1 | 0.5 | 3335 | 9.4E-03 | 0.44 |
| CA1 | 0.5 | 3194 | 9.6E-03 | 0.44 |
| ITGA2B | 0.5 | 1066 | 9.7E-03 | 2.27 |
| BTK | 0.6 | 550 | 9.8E-03 | 0.42 |
| ESD | 0.6 | 14095 | 9.9E-03 | 0.42 |
| Fibronectin | 0.4 | 9884 | 1.0E-02 | 2.35 |
| PGAM1 | 0.5 | 294 | 1.0E-02 | 2.21 |
| FGR | 0.4 | 279 | 1.0E-02 | 0.46 |
| IL13RA1 | 0.6 | 7319 | 1.0E-02 | 2.17 |
| CDH1 | 0.7 | 38432 | 1.0E-02 | 2.19 |
| IFNB1 | 0.6 | 552 | 1.0E-02 | 0.44 |
| EREG | 0.5 | 642 | 1.1 E-02 | 2.26 |
| ARSA | 0.4 | 2153 | 1.1 E-02 | 2.24 |
| SNCA | 0.5 | 764 | 1.1 E-02 | 0.46 |
| PRKCQ | 0.3 | 921 | 1.1 E-02 | 0.43 |
| FGF23 | 0.4 | 415 | 1.1 E-02 | 2.28 |
| MAPK8 | 0.6 | 1472 | 1.2E-02 | 0.42 |
| CRP | 0.5 | 71116 | 1.2E-02 | 2.18 |
| PI3 | 0.7 | 15648 | 1.2E-02 | 2.23 |
| TNFRSF10D | 0.5 | 1632 | 1.2E-02 | 2.20 |
| AKT2 | 0.3 | 11829 | 1.2E-02 | 0.45 |
| EFNA5 | 0.3 | 28667 | 1.2E-02 | 2.50 |
| KYNU | 0.4 | 2574 | 1.2E-02 | 0.46 |
| PRKAA2 | 0.6 | 3187 | 1.3E-02 | 0.43 |
| CCL28 | 0.5 | 3982 | 1.3E-02 | 2.18 |
| POSTN | 0.3 | 6786 | 1.3E-02 | 2.55 |
| DDX19B | 0.6 | 6771 | 1.3E-02 | 0.43 |
| DUSP3 | 0.5 | 2093 | 1.3E-02 | 0.46 |
| CCL1 | 0.5 | 1570 | 1.3E-02 | 2.20 |
| LILRB1 | 0.5 | 6174 | 1.3E-02 | 2.15 |
| KPNB1 | 0.5 | 43836 | 1.3E-02 | 0.46 |
| RAD51 | 0.3 | 1816 | 1.3E-02 | 2.86 |
| CTSF | 0.4 | 967 | 1.4E-02 | 0.48 |
| HGF | 0.7 | 5059 | 1.4E-02 | 2.15 |
| HSPB1 | 0.7 | 11609 | 1.4E-02 | 0.39 |
| PRKCZ | 0.4 | 5865 | 1.4E-02 | 0.46 |
| IL17RC | 0.4 | 1182 | 1.4E-02 | 2.20 |
| CK-MB | 0.7 | 282 | 1.4E-02 | 0.39 |
| IL1RN | 0.5 | 11394 | 1.4E-02 | 2.13 |
| CKM | 0.7 | 304 | 1.4E-02 | 0.40 |
| IL27RA | 0.7 | 898 | 1.4E-02 | 2.17 |
| EFNA4 | 0.3 | 5401 | 1.5E-02 | 2.49 |
| ICOS | 0.6 | 1086 | 1.5E-02 | 0.44 |
| IL22RA1 | 0.3 | 624 | 1.5E-02 | 0.46 |
| C5 | 0.5 | 4841 | 1.6E-02 | 2.13 |
| TNFRSF13C | 0.6 | 1954 | 1.6E-02 | 0.43 |
| LYNB | 0.5 | 9420 | 1.6E-02 | 0.47 |
| NOG | 0.7 | 2461 | 1.6E-02 | 2.13 |
| CCNB1 | 0.3 | 273 | 1.6E-02 | 2.40 |
| IL27 | 0.5 | 443 | 1.6E-02 | 2.07 |
| Thrombin | 0.7 | 7164 | 1.6E-02 | 2.08 |
| TNC | 0.5 | 34094 | 1.6E-02 | 2.10 |
| SEMA6A | 0.7 | 14145 | 1.6E-02 | 2.19 |
| DYNLL1 | 0.4 | 418 | 1.6E-02 | 0.47 |
| GP6 | 0.6 | 2422 | 1.7E-02 | 2.05 |
| GDF15 | 0.4 | 2812 | 1.7E-02 | 2.13 |
| PDE5A | 0.6 | 2020 | 1.7E-02 | 0.45 |
| RPS3 | 0.3 | 1262 | 1.7E-02 | 0.46 |
| CD59 | 0.3 | 1826 | 1.7E-02 | 2.44 |
| C6 | 0.3 | 44696 | 1.7E-02 | 2.60 |
| GDF5 | 0.3 | 1217 | 1.7E-02 | 2.38 |
| HDAC8 | 0.5 | 2054 | 1.7E-02 | 0.47 |
| CNDP1 | 0.5 | 1652 | 1.8E-02 | 0.48 |
| GSK3A | 0.5 | 4653 | 1.8E-02 | 0.47 |
| CDK8 | 0.3 | 828 | 1.8E-02 | 0.48 |
| HSD17B10 | 0.3 | 1833 | 1.8E-02 | 0.47 |
| EPHB6 | 0.5 | 4319 | 1.8E-02 | 2.10 |
| HS6ST1 | 0.4 | 1937 | 1.8E-02 | 2.23 |
| ICAM3 | 0.4 | 934 | 1.9E-02 | 0.48 |
| CXCL3 | 0.7 | 721 | 1.9E-02 | 0.41 |
| EPHA5 | 0.6 | 1455 | 1.9E-02 | 0.47 |
| PRKCB | 0.7 | 241 | 1.9E-02 | 0.41 |
| CDK5 | 0.3 | 761 | 2.0E-02 | 2.56 |
| GFRA1 | 0.7 | 1834 | 2.0E-02 | 2.08 |
| VWF | 0.3 | 7197 | 2.0E-02 | 2.33 |
| SGTA | 0.5 | 1348 | 2.0E-02 | 0.48 |
| TNFSF15 | 0.3 | 12777 | 2.0E-02 | 2.26 |
| STAT1 | 0.5 | 17913 | 2.1 E-02 | 0.48 |
| POR | 0.4 | 7263 | 2.1 E-02 | 0.50 |
| PPP3CA | 0.5 | 1129 | 2.1 E-02 | 0.48 |
| PON1 | 0.3 | 205 | 2.2E-02 | 0.48 |
| IGHA1 | 0.5 | 4665 | 2.2E-02 | 2.04 |
| EFNA2 | 0.3 | 4547 | 2.2E-02 | 2.30 |
| PSMD7 | 0.6 | 797 | 2.2E-02 | 0.46 |
| MMP14 | 0.3 | 942 | 2.2E-02 | 2.39 |
| Activated Protein C | 0.6 | 1408 | 2.2E-02 | 2.00 |
| APP | 0.5 | 23177 | 2.2E-02 | 2.01 |
| IL6 | 0.6 | 60069 | 2.2E-02 | 2.05 |
| CSF3 | 0.4 | 2159 | 2.2E-02 | 2.18 |
| TXNDC12 | 0.7 | 3443 | 2.2E-02 | 1.99 |
| PTK2 | 0.5 | 225 | 2.3E-02 | 0.49 |
| TNFRSF18 | 0.3 | 1030 | 2.3E-02 | 2.51 |
| HPX | 0.6 | 734 | 2.3E-02 | 1.98 |
| SHH | 0.5 | 496 | 2.3E-02 | 2.00 |
| KIRREL3 | 0.7 | 340 | 2.3E-02 | 2.07 |
| LRP1 | 0.5 | 4080 | 2.4E-02 | 2.06 |
| MED1 | 0.7 | 25405 | 2.4E-02 | 2.06 |
| NRXN3 | 0.4 | 1790 | 2.4E-02 | 2.06 |
| CSF2 | 0.5 | 389 | 2.4E-02 | 0.49 |
| CXCL5 | 0.4 | 425 | 2.4E-02 | 0.50 |
| C2 | 0.7 | 2978 | 2.4E-02 | 1.97 |
| NLGN4X | 0.5 | 6275 | 2.4E-02 | 2.02 |
| ACVR1 B | 0.4 | 500 | 2.4E-02 | 0.49 |
| TPT1 | 0.6 | 19080 | 2.4E-02 | 0.47 |
| SELP | 0.4 | 6221 | 2.5E-02 | 2.10 |
| CTSS | 0.4 | 2017 | 2.5E-02 | 2.10 |
| TBP | 0.6 | 1587 | 2.5E-02 | 0.47 |
| IDE | 0.6 | 2213 | 2.5E-02 | 0.48 |
| PTPN6 | 0.7 | 2198 | 2.5E-02 | 0.46 |
| LMAN2 | 0.6 | 7956 | 2.6E-02 | 1.96 |
| HAVCR2 | 0.4 | 24745 | 2.6E-02 | 2.04 |
| Corticotropin-lipotropin | 0.7 | 1020 | 2.6E-02 | 2.11 |
| CD63 | 0.6 | 567 | 2.6E-02 | 0.47 |
| RPSA | 0.3 | 3221 | 2.6E-02 | 2.24 |
| MMP3 | 0.6 | 16961 | 2.6E-02 | 1.95 |
| TGFB1 | 0.3 | 2063 | 2.6E-02 | 0.49 |
| EPHB2 | 0.3 | 48678 | 2.6E-02 | 2.16 |
| TNF-b | 0.6 | 641 | 2.7E-02 | 0.47 |
| RPS7 | 0.4 | 9423 | 2.7E-02 | 2.01 |
| STK16 | 0.5 | 1412 | 2.7E-02 | 2.00 |
| ADSL | 0.6 | 9055 | 2.7E-02 | 0.48 |
| CXCL16 | 0.3 | 26188 | 2.7E-02 | 2.32 |
| MMP9 | 0.4 | 5274 | 2.7E-02 | 2.08 |
| PRDX1 | 0.7 | 6471 | 2.8E-02 | 0.43 |
| ICAM2 | 0.4 | 1571 | 2.8E-02 | 0.52 |
| KLK12 | 0.7 | 3117 | 2.9E-02 | 1.96 |
| FLRT2 | 0.6 | 133815 | 2.9E-02 | 1.93 |
| FLRT3 | 0.4 | 12426 | 2.9E-02 | 2.07 |
| NID2 | 0.5 | 8496 | 2.9E-02 | 1.99 |
| CHEK2 | 0.6 | 200 | 2.9E-02 | 0.48 |
| IL1R1 | 0.5 | 18629 | 2.9E-02 | 1.97 |
| IL16 | 0.4 | 449 | 2.9E-02 | 0.52 |
| IL23R | 0.3 | 2187 | 2.9E-02 | 0.50 |
| PDK1 | 0.3 | 993 | 2.9E-02 | 0.50 |
| HK2 | 0.7 | 7672 | 2.9E-02 | 0.46 |
| CASP2 | 0.3 | 300 | 2.9E-02 | 0.49 |
| BCL2 | 0.4 | 1838 | 2.9E-02 | 0.51 |
| PROS1 | 0.3 | 2377 | 2.9E-02 | 2.22 |
| MET | 0.6 | 5253 | 2.9E-02 | 1.96 |
| OAS1 | 0.6 | 863 | 2.9E-02 | 0.48 |
| FSTL3 | 0.6 | 31071 | 3.0E-02 | 1.94 |
| LTF | 0.3 | 26748 | 3.0E-02 | 0.49 |
| SUMO2 | 0.4 | 8384 | 3.0E-02 | 0.52 |
| CD300C | 0.7 | 12171 | 3.0E-02 | 2.05 |
| EI F4A3 | 0.5 | 846 | 3.0E-02 | 0.50 |
| XRCC6 | 0.7 | 2751 | 3.0E-02 | 0.44 |
| SECTM1 | 0.3 | 1868 | 3.0E-02 | 0.50 |
| Ubiquitin | 0.5 | 1950 | 3.0E-02 | 0.51 |
| CFP | 0.3 | 120690 | 3.0E-02 | 2.23 |
| ARSB | 0.7 | 1139 | 3.0E-02 | 1.98 |
| EPHA2 | 0.7 | 13292 | 3.1 E-02 | 2.00 |
| NAAA | 0.6 | 6687 | 3.1 E-02 | 0.49 |
| ARG1 | 0.6 | 1004 | 3.1 E-02 | 0.48 |
| PKM | 0.4 | 14219 | 3.1 E-02 | 0.52 |
| PRDX6 | 0.5 | 509 | 3.1 E-02 | 0.51 |
| MATN2 | 0.4 | 23453 | 3.1 E-02 | 2.01 |
| CD163 | 0.3 | 10002 | 3.1 E-02 | 2.16 |
| ASGR1 | 0.5 | 5494 | 3.1 E-02 | 1.93 |
| COLEC12 | 0.4 | 9502 | 3.1 E-02 | 2.02 |
| MRC1 | 0.4 | 7807 | 3.1 E-02 | 2.02 |
| HAT1 | 0.7 | 327 | 3.1 E-02 | 0.45 |
| KLK11 | 0.3 | 79027 | 3.2E-02 | 2.14 |
| ADGRE5 | 0.5 | 1458 | 3.2E-02 | 1.94 |
| MAPK3 | 0.6 | 41710 | 3.2E-02 | 0.49 |
| ALB | 0.4 | 8122 | 3.2E-02 | 0.53 |
| ADAMTS13 | 0.3 | 2835 | 3.2E-02 | 2.20 |
| Ubiquitin+1 | 0.6 | 2968 | 3.2E-02 | 0.49 |
| GDI2 | 0.7 | 35171 | 3.2E-02 | 0.44 |
| BPI | 0.5 | 1764 | 3.3E-02 | 0.52 |
| PTN | 0.3 | 860 | 3.3E-02 | 0.50 |
| SFTPD | 0.4 | 91929 | 3.3E-02 | 0.52 |
| SOD1 | 0.4 | 2156 | 3.3E-02 | 0.52 |
| C3d | 0.7 | 24557 | 3.3E-02 | 1.92 |
| PLAUR | 0.5 | 42568 | 3.3E-02 | 1.93 |
| TIMP1 | 0.6 | 4914 | 3.3E-02 | 1.91 |
| ANXA5 | 0.6 | 1242 | 3.3E-02 | 0.49 |
| TNFRSF8 | 0.5 | 4085 | 3.4E-02 | 1.94 |
| D-dimer | 0.4 | 6655 | 3.4E-02 | 1.93 |
| IFNL1 | 0.7 | 661 | 3.4E-02 | 0.44 |
| ROR1 | 0.4 | 1168 | 3.5E-02 | 2.02 |
| ADAMTS1 | 0.4 | 648 | 3.5E-02 | 0.52 |
| NCK1 | 0.4 | 1981 | 3.5E-02 | 2.00 |
| CDK1 | 0.6 | 1108 | 3.5E-02 | 0.49 |
| VTA1 | 0.5 | 14660 | 3.6E-02 | 0.51 |
| HSPD1 | 0.3 | 86278 | 3.6E-02 | 0.51 |
| SRC | 0.3 | 1159 | 3.6E-02 | 0.51 |
| PPID | 0.7 | 674 | 3.6E-02 | 0.45 |
| SERPING1 | 0.4 | 16986 | 3.7E-02 | 2.00 |
| PAK7 | 0.3 | 692 | 3.7E-02 | 0.50 |
| MAP3K7 | 0.5 | 1211 | 3.7E-02 | 0.52 |
| ANGPT2 | 0.3 | 1512 | 3.8E-02 | 2.14 |
| PPIA | 0.4 | 115742 | 3.8E-02 | 0.53 |
| SNAP25 | 0.5 | 763 | 3.8E-02 | 0.53 |
| COL18A1 | 0.7 | 72245 | 3.8E-02 | 1.88 |
| UFC1 | 0.7 | 24953 | 3.8E-02 | 0.47 |
| SERPINA10 | 0.4 | 3705 | 3.8E-02 | 2.02 |
| CGB | 0.4 | 2613 | 3.9E-02 | 1.97 |
| LILRB2 | 0.6 | 14258 | 3.9E-02 | 1.90 |
| IGFBP4 | 0.5 | 3082 | 3.9E-02 | 0.52 |
| THBS2 | 0.7 | 251952 | 3.9E-02 | 1.97 |
| COMMD7 | 0.5 | 7590 | 4.0E-02 | 0.53 |
| PPIB | 0.7 | 2865 | 4.0E-02 | 0.47 |
| TNFRSF12A | 0.3 | 391 | 4.0E-02 | 2.14 |
| TNFSF8 | 0.3 | 13945 | 4.0E-02 | 2.08 |
| TPM2 | 0.7 | 940 | 4.0E-02 | 1.88 |
| PIAS4 | 0.6 | 649 | 4.0E-02 | 0.50 |
| PEBP1 | 0.6 | 7645 | 4.0E-02 | 0.51 |
| EPHA1 | 0.4 | 17832 | 4.1 E-02 | 1.92 |
| TEK | 0.3 | 1462 | 4.1 E-02 | 2.12 |
| SCG F-beta | 0.6 | 6527 | 4.1 E-02 | 1.90 |
| KRT18 | 0.7 | 751 | 4.1 E-02 | 0.47 |
| CRLF1 | 0.3 | 299 | 4.2E-02 | 2.13 |
| EFNB2 | 0.7 | 39070 | 4.2E-02 | 1.88 |
| RAN | 0.6 | 1404 | 4.2E-02 | 0.51 |
| AMH | 0.6 | 9209 | 4.2E-02 | 1.86 |
| LAYN | 0.6 | 7401 | 4.2E-02 | 1.90 |
| CCL5 | 0.5 | 215 | 4.2E-02 | 0.53 |
| SPOCK1 | 0.6 | 3203 | 4.2E-02 | 0.51 |
| DPP7 | 0.7 | 1871 | 4.3E-02 | 0.48 |
| AKT1 | 0.4 | 5728 | 4.3E-02 | 0.54 |
| SCARB2 | 0.7 | 453 | 4.3E-02 | 0.47 |
| CFB | 0.3 | 21479 | 4.4E-02 | 2.10 |
| VEGF | 0.7 | 19785 | 4.4E-02 | 1.82 |
| IL22RA2 | 0.4 | 2487 | 4.5E-02 | 1.91 |
| UBB | 0.7 | 1672 | 4.5E-02 | 0.48 |
| FUT3 | 0.7 | 746 | 4.5E-02 | 1.96 |
| LGALS2 | 0.7 | 1580 | 4.5E-02 | 0.48 |
| FGF16 | 0.7 | 3942 | 4.5E-02 | 0.48 |
| SERPIND1 | 0.4 | 1586 | 4.5E-02 | 1.98 |
| GPC3 | 0.6 | 12854 | 4.6E-02 | 1.86 |
| IFNGR1 | 0.3 | 1203 | 4.6E-02 | 2.05 |
| SMAD3 | 0.5 | 10911 | 4.6E-02 | 0.54 |
| SCGF-alpha | 0.6 | 19274 | 4.6E-02 | 1.87 |
| AK1 | 0.4 | 6995 | 4.7E-02 | 0.54 |
| SH2D1A | 0.7 | 4908 | 4.7E-02 | 0.48 |
| AKR7A2 | 0.7 | 906 | 4.7E-02 | 0.48 |
| CDON | 0.7 | 10310 | 4.7E-02 | 0.49 |
| AIF1 | 0.7 | 11131 | 4.7E-02 | 0.47 |
| FGF6 | 0.7 | 547 | 4.7E-02 | 0.48 |
| CTGF | 0.7 | 1740 | 4.7E-02 | 0.48 |
| IL3RA | 0.7 | 1633 | 4.7E-02 | 0.48 |
| PDXP | 0.7 | 7954 | 4.7E-02 | 0.48 |
| UBE2L3 | 0.7 | 4224 | 4.7E-02 | 0.48 |
| C4 | 0.5 | 68233 | 4.7E-02 | 1.88 |
| CD84 | 0.5 | 16969 | 4.7E-02 | 1.85 |
| EFNA3 | 0.7 | 2428 | 4.7E-02 | 1.83 |
| ERBB1 | 0.3 | 14099 | 4.8E-02 | 2.15 |
| LUM | 0.3 | 2585 | 4.8E-02 | 2.02 |
| GDF-11 | 0.6 | 628 | 4.8E-02 | 1.84 |
| ZAP70 | 0.4 | 5820 | 4.8E-02 | 1.98 |
| ENPP7 | 0.7 | 1178 | 4.8E-02 | 1.85 |
| NID1 | 0.6 | 1715 | 4.8E-02 | 1.81 |
| PGF | 0.3 | 1536 | 4.9E-02 | 2.07 |
| KLRF1 | 0.7 | 206 | 4.9E-02 | 0.51 |
| ICAM5 | 0.6 | 1116 | 4.9E-02 | 1.84 |
| IGHM | 0.6 | 2067 | 4.9E-02 | 0.52 |
| PLA2G2A | 0.4 | 13525 | 4.9E-02 | 0.56 |
| HPV E7 Type18 | 0.3 | 1394 | 5.0E-02 | 2.11 |
| UBE2N | 0.7 | 7142 | 5.0E-02 | 0.48 |
| C10orf54 | 0.6 | 1131 | 5.0E-02 | 0.51 |

Table 2 shows 20 examples of the best performing combinations of type 1 and type 2 signatures yielding the highest possible percentage of predictions in descending order (>85%) with 0% false predictions.

**Table 2: Possible combinations of type 1 and type 2 signatures yielding the highest possible percentage of predictions (>85%) and 0% false predictions (POM C=Corticotropin-lipotropin).**

| **Combi ID** | **Signature 1** | **Signature 2** | **% predicted** | **% false** |
|---|---|---|---|---|
| 1 | HSPA1A, BCAM, DKK1, B4, DPP7, LMAN2, CTSS, RPSA, SELP | CAPG, TNFAIP6, LCK, REG1A, CTSZ, ARSA, RPS7, CNTN2, ARSB | 85.7 | 0 |
| 983 | HSPA1A, BCAM, DKK1, POMC, DPP7, LMAN2, CTSS, CD59, LTF | CAPG, TNFAIP6, LCK, REG1A, CTSZ, ARSA, RPS7, IGHM, HGF | 85.7 | 0 |
| 981 | HSPA1A, BCAM, DKK1, POMC, DPP7, LMAN2, CTSS, CD59, LTF | CAPG, TNFAIP6, LCK, REG1A, CTSZ, ARSA, RPS7,KLRF1,IL13RA1 | 85.7 | 0 |
| 980 | HSPA1A, BCAM, DKK1, POMC, DPP7, LMAN2, CTSS, CD59, LTF | CAPG, TNFAIP6, LCK, REG1A, CTSZ, ARSA, RPS7, IGFBP4, LTF | 85.7 | 0 |
| 979 | HSPA1A, BCAM, DKK1, POMC, DPP7, LMAN2, CTSS, CD59, LTF | CAPG, TNFAIP6, LCK, REG1A, CTSZ, ARSA, RPS7, IGFBP4, ARSB | 85.7 | 0 |
| 978 | HSPA1A, BCAM, DKK1, POMC, DPP7, LMAN2, CTSS, CD59, LTF | CAPG, TNFAIP6, LCK, REG1A, CTSZ, ARSA, RPS7, CD27, CRLF1 | 85.7 | 0 |
| 977 | HSPA1A, BCAM, DKK1, POMC, DPP7, LMAN2, CTSS, CD59, LTF | CAPG, TNFAIP6, LCK, REG1A, CTSZ, ARSA, RPS7, CNTN2, ARSB | 85.7 | 0 |
| 976 | HSPA1A, BCAM, DKK1, POMC, DPP7, LMAN2, CTSS, CD59, IL36A | CAPG, TNFAIP6, LCK, REG1A, CTSZ, ARSA, RPS7, CRLF1, C5 | 85.7 | 0 |
| 975 | HSPA1A, BCAM, DKK1, POMC, DPP7, LMAN2, CTSS, CD59, IL36A | CAPG, TNFAIP6, LCK, REG1A, CTSZ, ARSA, RPS7, IGHM, HGF | 85.7 | 0 |
| 974 | HSPA1A, BCAM, DKK1, POMC, DPP7, LMAN2, CTSS, CD59, IL36A | CAPG, TNFAIP6, LCK, REG1A, CTSZ, ARSA, RPS7, MMP14, PTN | 85.7 | 0 |
| 973 | HSPA1A, BCAM, DKK1, POMC, DPP7, LMAN2, CTSS, CD59, IL36A | CAPG, TNFAIP6, LCK, REG1A, CTSZ, ARSA, RPS7,KLRF1,IL13RA1 | 85.7 | 0 |
| 972 | HSPA1A, BCAM, DKK1, POMC, DPP7, LMAN2, CTSS, CD59, IL36A | CAPG, TNFAIP6, LCK, REG1A, CTSZ, ARSA, RPS7, IGFBP4, LTF | 85.7 | 0 |
| 971 | HSPA1A, BCAM, DKK1, POMC, DPP7, LMAN2, CTSS, CD59, IL36A | CAPG, TNFAIP6, LCK, REG1A, CTSZ, ARSA, RPS7, IGFBP4, ARSB | 85.7 | 0 |
| 970 | HSPA1A, BCAM, DKK1, POMC, DPP7, LMAN2, CTSS, CD59, IL36A | CAPG, TNFAIP6, LCK, REG1A, CTSZ, ARSA, RPS7, CD27, CRLF1 | 85.7 | 0 |
| 969 | HSPA1A, BCAM, DKK1, POMC, DPP7, LMAN2, CTSS, CD59, IL36A | CAPG, TNFAIP6, LCK, REG1A, CTSZ, ARSA, RPS7, CNTN2, ARSB | 85.7 | 0 |
| 968 | HSPA1A, BCAM, DKK1, POMC, DPP7, LMAN2, CTSS, CD59, HSPD1 | CAPG, TNFAIP6, LCK, REG1A, CTSZ, ARSA, RPS7, CRLF1, C5 | 85.7 | 0 |
| 967 | HSPA1A, BCAM, DKK1, POMC, DPP7, LMAN2, CTSS, CD59, HSPD1 | CAPG, TNFAIP6, LCK, REG1A, CTSZ, ARSA, RPS7, IGHM, HGF | 85.7 | 0 |
| 966 | HSPA1A, BCAM, DKK1, POMC, DPP7, LMAN2, CTSS, CD59, HSPD1 | CAPG, TNFAIP6, LCK, REG1A, CTSZ, ARSA, RPS7, MMP14, PTN | 85.7 | 0 |
| 965 | HSPA1A, BCAM, DKK1, POMC, DPP7, LMAN2, CTSS, CD59, HSPD1 | CAPG, TNFAIP6, LCK, REG1A, CTSZ, ARSA, RPS7,KLRF1,IL13RA1 | 85.7 | 0 |
| 964 | HSPA1A, BCAM, DKK1, POMC, DPP7, LMAN2, CTSS, CD59, HSPD1 | CAPG, TNFAIP6, LCK, REG1A, CTSZ, ARSA, RPS7, IGFBP4, LTF | 85.7 | 0 |

In order to execute the method according to claim 1 a device (e.g. test kit) can be used. This device is a test kit for determining at least the concentration of the following markers in a biological sample like as peritoneal fluid from a subject with proven OC: HSPA1A, BCAM, and DKK1 (markers identifying all long-term relapse-free survivors) and CAPG, LCK, and TNFAIP6 (markers identifying all short-term relapse-free survivors).

The device for the determination of the prognosis of an OC in a biological sample from an individual with diagnosed OC according to claim 5 comprises at least following components:
- a measuring unit for measuring the concentration of at least the markers HSPA1A, BCAM, and DKK1 (markers identifying all long-term relapse-free survivors) and CAPG, LCK and TNFAIP6 (markers identifying all short-term relapse-free survivors) in the biological sample of said individual whereby this unit is capable to forward the measured concentrations to a data processing unit;
- a data processing unit which receives the measured concentrations from the measuring unit and which executes the steps (iii) to (xii) of claim 1 and forwards the results to the output unit;
- an input unit for data entry which is connectable to a database for the input of at least the threshold values and which forwards data to the data processing unit;
- an output unit for data output which indicates a short-term relapse-free survival in said individual if the multi-marker score from step (xii) of claim 1 is 0.5 or higher and indicating a long-term relapse-free survival in said individual if the multi-marker score from step (xii) of claim 1 is below 0.5.
- A database or a connection to a database for at least the threshold values: This database preferable is implemented in the data processing unit. In an embodiment, this database is connected to the data processing unit via the input unit.

The concentration of these markers can be measured by ELISA-based techniques, mass spectrometry, antibody- or aptamer-based microarrays or any other suitable quantification method, however preferably a SOMAscan® Proteomic Assay.

The invention also comprises a computer software to perform the method according to claim 1. In a preferred embodiment, this computer software is implemented in the data processing unit and performs the steps (iii) to (xii) of claim 1.

### Embodiments

### 1. Determination of markers in biological samples like as peritoneal fluid as markers for the prognosis of OC

The inventors applied the SOMAscan® Proteomic Assay for a targeted proteomic approach to determine reliable markers for the prognosis of OC. They first measured the concentration of 1,305 potential markers in peritoneal fluid samples of 70 patients with high-grade serous OC and in plasma samples of 20 patients with high-grade serous OC (OC-plasma). The inventors also measured the concentration of the same 1,305 potential markers in plasma samples from 10 subjects with non-malignant diseases (N-plasma).

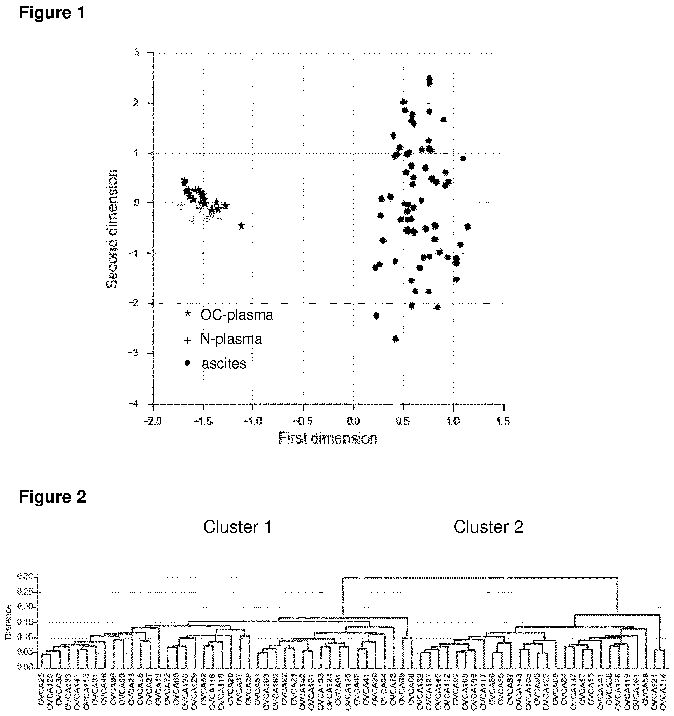

Afterward the inventors performed a principal component analysis (PCA) of these samples with normalized SOMAscan units, which yielded a complete separation of all plasma samples (OC-plasma and N-plasma) from all peritoneal fluid samples. Figure 1 shows these results. In a next step, the inventors carried out analyses to identify the markers, which are upregulated in peritoneal fluid samples.

### 2. Identification of markers in peritoneal fluid, which are consistent with OC biology

The inventors defined 779 markers out of the 1,305 potential markers with significantly higher concentrations in peritoneal fluid samples versus plasma samples (OC-plasma and N-plasma). The p-value is <0.05 by two-sided unpaired t-test and adjusted for multiple hypothesis testing by Benjamini-Hochberg correction. Of these 356 markers, 45 were at least 5-fold more abundant in peritoneal fluid samples relative to plasma samples. The marker IL6 shows the greatest difference with a fold change (FC) of 89.8 (see Table 3).

Hierarchical clustering of peritoneal fluid samples identified two clearly separable clusters (cluster 1 and 2 in Figure 2). While the levels of 373 markers were significantly higher in cluster 1 versus cluster 2, 406 markers showed the opposite pattern (Figure 3). Intriguingly, the vast majority of markers in cluster 1 is associated with a short RFS (relapse-free survival) of OC (n=115 markers versus 0 markers linked to a long RFS; see Figure 4). By contrast, most markers in cluster 2 are associated with a long RFS (n=135 markers versus 2 markers linked to a short RFS; see Figure 4).

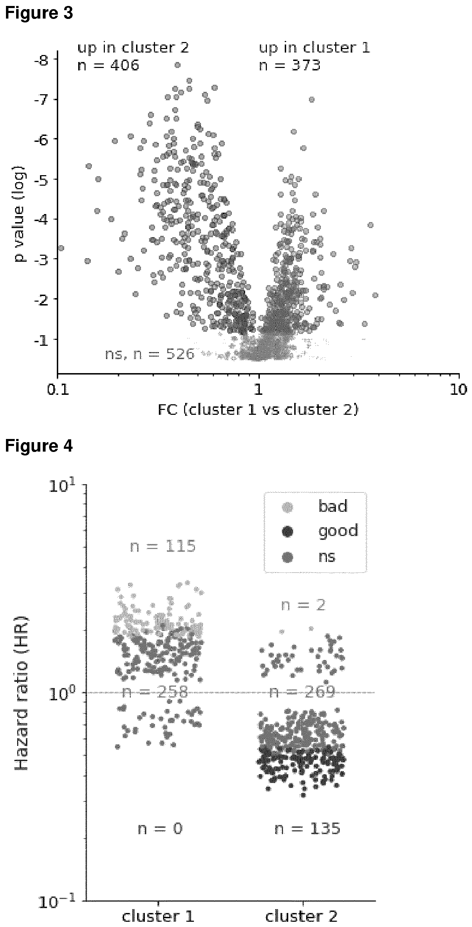

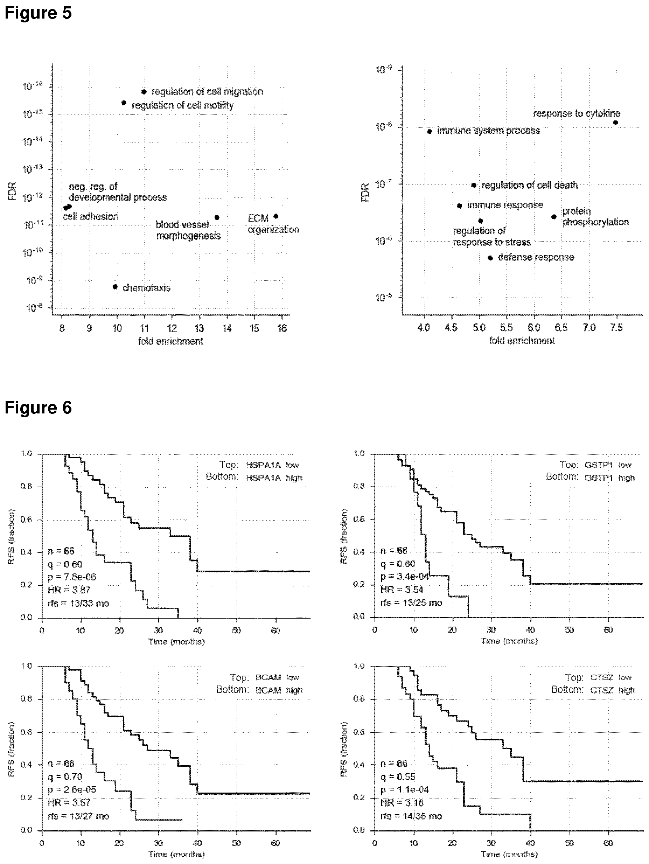

The inventors performed a PANTHER gene ontology (GO) enrichment analysis in order to gain better insight into the function of the markers in the two different clusters. The inventors concentrated on RFS-associated markers. As shown in Figure 5, the markers in cluster 1 are predominantly associated with metastasis-associated biological processes, such as cell motility, migration, adhesion, extracellular matrix remodelling (ECM), and angiogenesis. This is consistent with the observed association of these markers with a short relapse-free survival time (RFS) (Figure 4). On the contrary, the markers in cluster 2 are mainly linked to immune regulation. Taken together with the RFS data in Figure 4, this shows that these markers play a role in the anti-tumour immune defence. This is consistent with the biological features known to determine the outcome of OC, i.e. peritoneal adhesion and invasion by cancer cells and a T-cell-mediated cytotoxic response.

**Table 3: Markers (proteins) found at ≥5-fold higher levels in peritoneal fluid (ascites) samples compared to plasma samples. FC: Fold change peritoneal fluid (ascites)/plasma. p adjusted: significance calculated by two-sided unpaired t-test and Benjamini-Hochberg correction.**

| **Protein/ Marker** | **FC** | **p adjusted** | **Description** |
|---|---|---|---|
| IL6 | 89.8 | 1.80E-09 | Interleukin 6 (Interferon beta 2) |
| TIMP1 | 32 | 1.70E-34 | TIMP Metallopeptidase Inhibitor 1 |
| CCDC80 | 21.7 | 9.70E-17 | Coiled-coil Domain Containing 80 |
| KLK11 | 15.3 | 1.50E-19 | Kallikrein-related Peptidase 11 |
| NBL1 | 15.1 | 1.10E-09 | Neuroblastoma 1, DAN Family BMP Antagonist |
| GPC3 | 12.8 | 8.90E-10 | Glypican 3 |
| HSPD1 | 11.4 | 2.00E-20 | Heat Shock 60kDa Protein 1 (Chaperonin) |
| RSPO3 | 11 | 3.90E-16 | R-spondin 3 |
| NLGN4X | 10.3 | 2.60E-10 | Neuroligin 4, X-linked |
| PFDN5 | 9.8 | 2.80E-13 | Prefoldin subunit 5 |
| VEGF121 | 9.4 | 3.00E-10 | Vascular Endothelial Growth Factor alpha |
| LTA4H | 9.1 | 8.40E-05 | Leukotriene A4 Hydrolase |
| ANXA2 | 9 | 7.40E-25 | Annexin A2 |
| HSP90AA1 | 8.7 | 1.10E-16 | Heat Shock Protein 90kDa alpha (cytosolic), class A member 1 |
| GAS1 | 8.4 | 1.40E-26 | Growth Arrest-specific 1 |
| XPNPEP1 | 7.8 | 1.30E-10 | X-Prolyl Aminopeptidase (Aminopeptidase P) 1, soluble |
| CXCL8 | 7.5 | 2.40E-08 | Interleukin 8 |
| STAT1 | 6.8 | 3.80E-09 | Signal Transducer and Activator of Transcription 1, 91kDa |
| RPS7 | 6.7 | 8.40E-10 | Ribosomal Protein S7 |
| NAMPT | 6.6 | 1.20E-05 | Nicotinamide Phosphoribosyltransferase |
| PRKACA | 6.5 | 2.50E-09 | Protein Kinase, cAMP-dependent, catalytic, alpha |
| THBS2 | 6.5 | 2.40E-25 | Thrombospondin 2 |
| HSPA1A | 6.4 | 1.60E-15 | Heat Shock 70kDa Protein 1A |
| KLK8 | 6.2 | 7.20E-11 | Kallikrein-related Peptidase 8 |
| LAMA1 | 6.1 | 8.10E-20 | Laminin, alpha 1 |
| SPOCK2 | 6 | 4.40E-05 | Sparc/Osteonectin, cwcv and kazal-like domains proteoglycan (Testican) 2 |
| PRSS22 | 6 | 1.10E-05 | Protease, Serine, 22 |
| HNRNPA2B1 | 6 | 8.50E-11 | Heterogeneous Nuclear Ribonucleoprotein A2/B1 |
| KPNB1 | 5.9 | 7.20E-14 | Karyopherin (Importin) beta 1 |
| CAPG | 5.8 | 1.70E-08 | Capping Protein (Actin Filament), Gelsolin-like |
| PRKCI | 5.7 | 2.10E-07 | Protein Kinase C, iota |
| DYNLRB1 | 5.7 | 8.80E-08 | Dynein, light chain, roadblock-type 1 |
| HSPB1 | 5.6 | 3.40E-09 | Heat Shock 27kDa Protein 1 |
| SCGF-alpha | 5.5 | 1.50E-12 | C-Type Lectin Domain Containing 11A (CLEC11A) |
| ISG15 | 5.4 | 1.60E-08 | ISG15 Ubiquitin-like Modifier |
| PRKCZ | 5.4 | 5.10E-09 | Protein Kinase C, zeta |
| ANGPT2 | 5.3 | 1.10E-16 | Angiopoietin 2 |
| Thrombin | 5.3 | 1.20E-07 | Thrombin |
| EIF4G2 | 5.3 | 3.40E-09 | Eukaryotic Translation Initiation Factor 4 gamma, 2 |
| EIF4H | 5.2 | 1.10E-10 | Eukaryotic Translation Initiation Factor 4H |
| TNFSF8 | 5.1 | 2.20E-20 | Tumor Necrosis Factor (Ligand) superfamily, member 8 |
| CXCL13 | 5 | 8.20E-09 | Chemokine (C-X-C motif) ligand 13 |
| HNRNPAB | 5 | 2.50E-05 | Heterogeneous Nuclear Ribonucleoprotein A/B |
| TPI1 | 5 | 4.20E-10 | Triosephosphate Isomerase 1 |

Taken together the inventors found that the marker concentrations in peritoneal fluid samples determined by SOMAscan® Proteomic Assay parallel the biology and outcome of OC in individual patients. Thus, the marker concentrations in peritoneal fluid samples provide prognostic tools to assess the expected clinical course in an OC-patient.

### 3. Association of individual markers in peritoneal fluid samples with relapse-free survival

The inventors determined the association of all markers in peritoneal fluid samples determined by SOMAscan® Proteomic Assay with the RFS of the same patients, in order to identify markers with a potentially prognostic value. A log rank p-value <0.05 was observed with 470 markers in peritoneal fluid (ascites) (Table 1). These 470 markers include
(i) Secreted proteins, such as cytokines, growth factors, hormones, proteases, and extracellular matrix components;
(ii) Membrane receptors, such as BMPR2, EPHA5, EPHB2, EPH6, ERBB1, LEPR, IL1R1, IL2RG, IL13R1, IL15RA, IL17RC, IL18R1, IL22RA1, IL27RA1, and PDGFRA. These membrane receptors could be secreted as soluble forms or present on extracellular vesicles (EVs);
(iii) Intracellular proteins, such as protein kinases. These intracellular proteins could be constituents of extracellular vesicles (EVs) or released from dying cells. A number of these intracellular proteins represent cell-type-specific components, such as LCK from T-cells. Therefore, they might be useful as surrogate markers for immune cells in the tumour environment, for example in ascites.

This analysis also identified numerous markers hitherto unknown associated with OC survival in either direction. Examples of Kaplan-Maier plots for several negatively associated markers are shown in Figure 6. Two of these associations have an exceptionally high significance (p <0.001), i.e., HSPA1A (heat shock protein 70) and BCAM (basal cell adhesion molecule/CD239). None of the markers associated with relapse-free survival are useful on their own to correctly distinguish patients with a poor or a good prognosis.

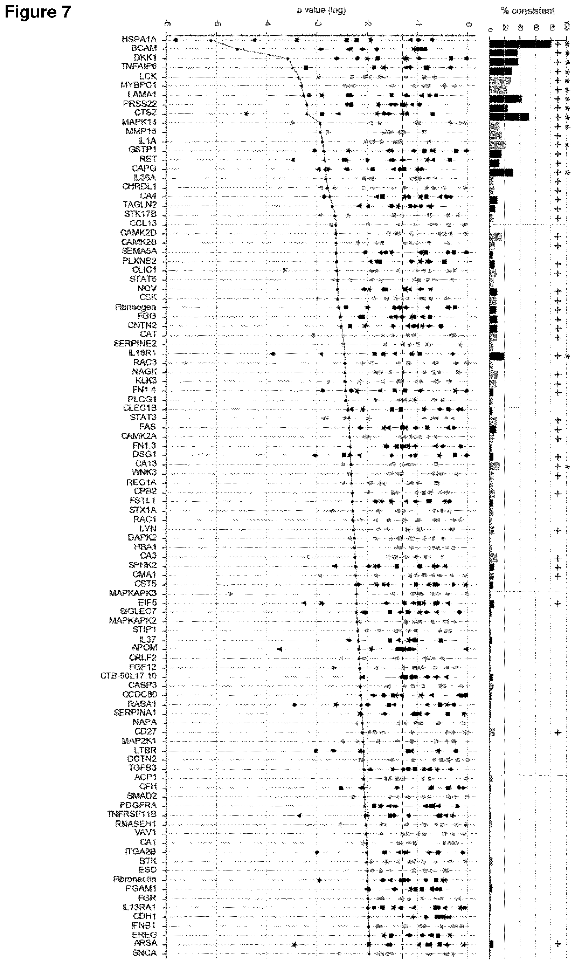

To test the robustness of potentially prognostic markers, the inventors split the patient cohort randomly into training and validation sets of equal size and repeated this simulation 500 times. Then the inventors determined the logrank p values of the resulting 1000 simulated samples and the percentage of simulations with both training and validation sets yielding significant p values (fitting simulations). The data for the top 100 markers are shown in Figure 7. This yielded 14 markers with a median p value <0.05 and the following 48 markers with a percentage of fitting simulations of at least 5%: ARSA, BCAM, CA13, CA3, CA4, CAMK2A, CAMK2B, CAMK2D, CAPG, CAT, CD27, CHRDL1, CLIC1, CMA1, CNTN2, CPB2, CSK, CTSZ, DKK1, DSG1, EIF5, FAS, FGG, FN1.4, Fibrinogen, GSTP1, HSPA1A, IL18R1, IL1A, IL36A, KLK3, LAMA1, LCK, LYN, MAPK14, MMP16, MYBPC1, NAGK, NOV, PLXNB2, PRSS22, RET, SPHK2, STAT3, STK17B, TAGLN2, TNFAIP6, and WNK3.

Table 4 shows a description of the abbreviations of the markers.

**Table 4: Alphabetical list of markers and their abbreviations**

| **Abbreviation** | **Description** | **Abbreviation** | **Description** |
|---|---|---|---|
| ARSA | Arylsulfatase A | IL1A | Interleukin 1 alpha |
| BCAM | basal cell adhesion molecule/CD239 | IL1R1 | Interleukin 1 Receptor Type 1 |
| BMPR2 | Bone Morphogenetic Protein Receptor 2 | IL2RG | Interleukin 2 Receptor Subunit gamma |
| BRCA1 | Breast Cancer 1 | IL6 | Interleukin 6 |
| CA3 | Carbonic Anhydrase 3 | IL10 | Interleukin 10 |
| CA4 | Carbonic Anhydrase 4 | IL13R1 | Interleukin 13 Receptor Type 1 |
| CA13 | Carbonic Anhydrase 13 | IL15RA | Interleukin 15 Receptor Subunit alpha |
| CA125 | Cancer Antigen 125 | IL17RC | Interleukin 17 Receptor Type C |
| CAMK2A | Calcium/Calmodulin-dependent Protein Kinase 2 alpha | IL18R1 | Interleukin 18 Receptor Type 1 |
| CAMK2B | Calcium/Calmodulin-dependent Protein Kinase 2 beta | IL22RA1 | Interleukin 22 Receptor Subunit alpha 1 |
| CAMK2D | Calcium/Calmodulin-dependent Protein Kinase 2 delta | IL27RA1 | Interleukin 27 Receptor Subunit alpha 1 |
| CAPG | Capping Protein (Actin Filament) Gelsolin-like | IL36A | Interleukin 36 alpha |
| CAT | Catalase | KLK3 | Kallikrein-related Peptidase 3 |
| CD27 | Cluster of Differentiation 27, Tumor Necrosis Factor Receptor Superfamily Member 7 | LAMA1 | Laminin alpha 1 |
| CHRDL1 | Chordin-like 1 | LCK | Lymphocyte-specific Tyrosine Protein Kinase |
| CLIC1 | Chloride Intracellular Channel Protein 1 | LEPR | Leptin Receptor |
| CMA1 | Chymase 1 | let-7b | Lethal 7b |
| CNTN2 | Contactin 2 | LYN | Tyrosine-Protein Kinase Lyn |
| CPB2 | Carboxypeptidase B2 | MAPK14 | Mitogen-activated Protein Kinase 14 |
| CSK | Tyrosine-Protein Kinase CSK | MMP16 | Matrix Metalloproteinase 16 |
| CTSZ | Cathepsin Z | MYBPC1 | Myosine-binding Protein C, slow type |
| CYCS | Mitochondrial Cytochrome C | NAGK | N-Acetyl-D-Glucosamine Kinase |
| DKK1 | Dickkopf-related Protein 1 | NOV | Nephroblastoma Overexpressed |
| DSG1 | Desmoglein-1 | PDGFRA | Platelet-derived growth factor receptor alpha |
| EIF5 | Eukaryotic Translation Initiation Factor 5 | PLXNB2 | Plexin-B2 |
| EPH6 | Ephrin 6 | PRSS22 | Protease, Serine, 22 |
| EPHA1 | Ephrin Type-A Receptor 1 | REG1A | Lithostatin 1 alpha |
| EPHA5 | Ephrin Type-A Receptor 5 | RET | RET Proto Oncogene |
| EPHB2 | Ephrin Type-B Receptor 2 | SPHK2 | Sphingosine Kinase 2 |
| ERBB1 | Epidermal Growth Factor Receptor 1 | STAT3 | Signal Transducer and Activator of Transcription 3 |
| FAS | Fas Receptor, Apoptosis Antigene 1 | STK17B | Serine/Threonine Kinase 17B |
| FG | Fibrinogen | TAGLN2 | Transgelin 2 |
| FGG | Fibrinogen Gamma Chain | TGF-beta | Transforming Growth Factor beta |
| FN1.4 | Fibronectin 1.4 | TNFAIP6 | Tumor Necrosis Factor-inducible Gene 6 Protein |
| GSTP1 | Glutathione S-Transferase P 1 | WNK3 | Serine/Threonine Kinase WNK3 7 |
| HIF1A | Hypoxia-inducible Factor 1 alpha | WT1 | Wilms Tumor Protein |
| HSPA1A | heat shock protein 1A 70kDa | | |

### 4. Identification of marker signatures for predicting the prognosis for patients with OC

None of the markers associated with relapse-free survival are useful on their own to correctly distinguish patients with a poor or favourable prognosis. The inventors therefore attempted to pursue a multivariate approach with the goal to develop prognostic marker signatures that are suitable for the recognition of short-term and long-term survivors. Toward this goal, the inventors tested combinations of markers in peritoneal fluid for their power to discriminate these patients.

Signatures are combinations of markers associated with relapse-free survival (p<0.05; Table 1) and present in peritoneal fluid at level >1724 SOMAscan® Proteomic Assay units in at least one sample (1724 is the median of all samples and markers). Signature score is the fraction of markers in a combination of markers (signature) above the best-fit threshold (Table 1) in individual patients. To determine the best-fit threshold of a given marker samples were split at different quantiles (between 0.3 and 0.7 in increments of 0.1) and the quantile (and corresponding concentration) yielding the lowest logrank p-value, i.e., the best fit, was determined. The inventors identified two types of signatures: type 1 signatures identify all long-term survivors; type 2 signatures identify all short-time survivors.

The markers associated with relapse-free survival (p<0.05; Table 1) and present in peritoneal fluid at >1724 SOMAscan® Proteomic Assay units in at least one sample were included in the approach to identify prognostic signatures by randomly combining these markers. Since it is virtually impossible to test all possible combinations of these markers, the inventors performed a step-wise analysis by first identifying a core signature with the most relevant markers followed by gradually increasing the length of this combination.

The bioinformatic approach for the definition of signatures discriminating patients with long and short relapse-free survival comprises the following steps:
- Analysis whether the level of each marker in the peritoneal fluid sample of individual patients is below or above the best-fit threshold (as in Table 1), and assignment of a score of one for all instances with a level below the threshold, otherwise a score of zero.
- Patient-wise addition of these scores and division by the number of markers in the respective signature to yield a signature score. A signature score >0.5 was considered a signature fit.
- Determination of the percentage of patients in the cohort of long-term and the cohort of short-term survivors with signature fits, and identification of signatures that identify 100% of either the long-term or the short-term relapse-free survivors (type 1 and type 2 signatures, respectively) with the highest possible specificity (i.e., the ability of a given type 1 signature to also correctly identify short-term survivors, or vice versa, the ability of a given type 2 signature to also correctly identify long-term survivors).

- Combining the best type 1 with the best type 2 signatures to determine the score for each patient: both signatures yielding a signature score >0.5: predicted short RFS; both signatures yielding a signature score <0.5: predicted long RFS; signature scores >0.5 and <0.5: prediction not possible.

This approach identified the markers BCAM, HSPA1A and DKK1 as the core type 1 signature, which was able to correctly identify 90.9% all long-term survivors and 67.6% short-term survivors.

Addition of for example Corticotropin-lipotropin and DPP7 yielded a type 1 signature (BCAM, HSPA1A, DKK1, Corticotropin-lipotropin, DPP7) that correctly identified 100% of long-term survivors and 76.5% of short-term survivors.

Further addition of for example LMAN2 and CTSS yielded a type 1 signature (BCAM, HSPA1A, DKK1, Corticotropin-lipotropin, DPP7, LMAN2, CTSS) that correctly identified 100% of long-term survivors and 82.4 % of short-term survivors. Further addition of for example RPSA and ARSB yielded a type 1 signature (BCAM, HSPA1A, DKK1, Corticotropin-lipotropin, DPP7, LMAN2, CTSS, RPSA, ARSB) correctly identified 100% of long-term survivors and 85.3 % of short-term survivors. For type 2 signatures the inventors identified the markers CAPG, LCK and TNFAIP6 as the core type 2 signature, which was able to correctly identify 91.2% of short-term survivors and 63.6% long-term survivors.

Addition of for example REG1A yielded a type 2 signature (CAPG, LCK, TNFAIP6, REG1A) that correctly identified 100% of short-term survivors and 59.1% of long-term survivors.

Further addition of for example CTSZ, ARSA and RPS7 yielded a type 2 signature (CAPG, LCK, TNFAIP6, REG1A, CTSZ, ARSA, RPS7) that correctly identified 100% of short-term survivors and 81.8% long-term survivors.

Further addition of for example CD27 and TNFAIP6 yielded a type 2 signature (CAPG, LCK, TNFAIP6, REG1A, CTSZ, ARSA, RPS7, CD27, CRLF1) that correctly identified 100% of short-term survivors and 86.4% long-term survivors.

Next, the inventors tested combinations of type 1 and 2 signatures for best performance, defined as a percentage of correctly identified short-term and long-term survivors of 100% (and consequently a percentage of false of 0%). To this end, they defined the following scoring system: First, those patients are identified, for which both signatures were consistent (i.e., above or below the threshold: signature score > or <0.5). Inconsistent instances were considered as outcome not predictable. For consistent instances, predictions were considered either "bad" for scores (added signature 1 and 2 scores) above 50% of the maximally possible score, or "good" for scores below 50% of the maximally possible score. The maximally possible score is the added length of both signatures. Combination of the two 3-marker core signatures correctly identified 64.4% of short-term and long-term survivors with only 7.1% false predictions. However, combinations of extended signatures identified more of short-term and long-term survivors with no false predictions. For example, 1464 combinations of type 1 and type 2 signatures, each combinations of 9 markers, correctly identified >85% of short-term and long-term survivors with no false predictions. Table 2 shows 20 examples of these combinations.

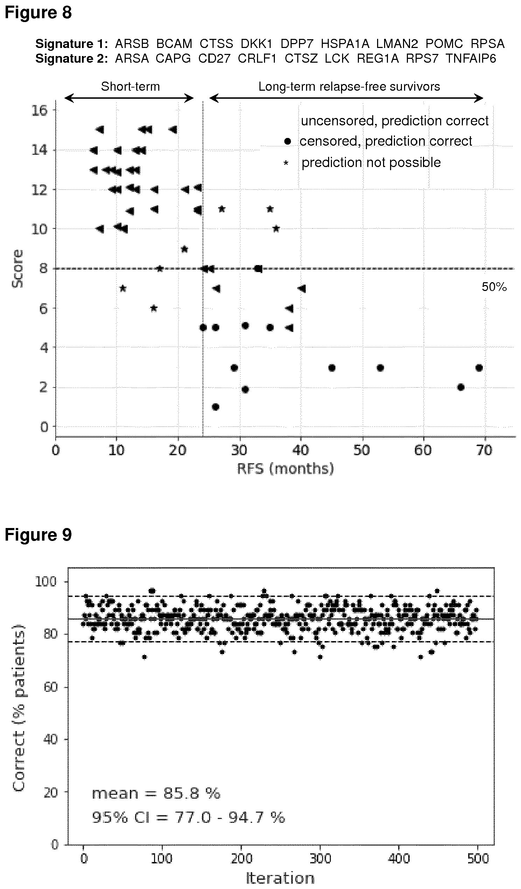

The result for one of these combinations of signatures (Combi ID: 1) is illustrated in detail in Figure 8. These signatures are composed of the following 18 proteins: BCAM, HSPA1A, DKK1, Corticotropin-lipotropin, DPP7, LMAN2, CTSS, RPSA, ARSB (type 1) and CAPG, LCK, TNFAIP6, REG1A, CTSZ, ARSA, RPS7, CD27, CRLF1 (type 2).

The robustness of this these combination of signatures was confirmed by the bootstrapping analysis in Figure 9, which yielded a median of 85.8% (95% CI = 77.0 - 94.7%) for the correct detection of short-term and long-term survivors with no false predictions.

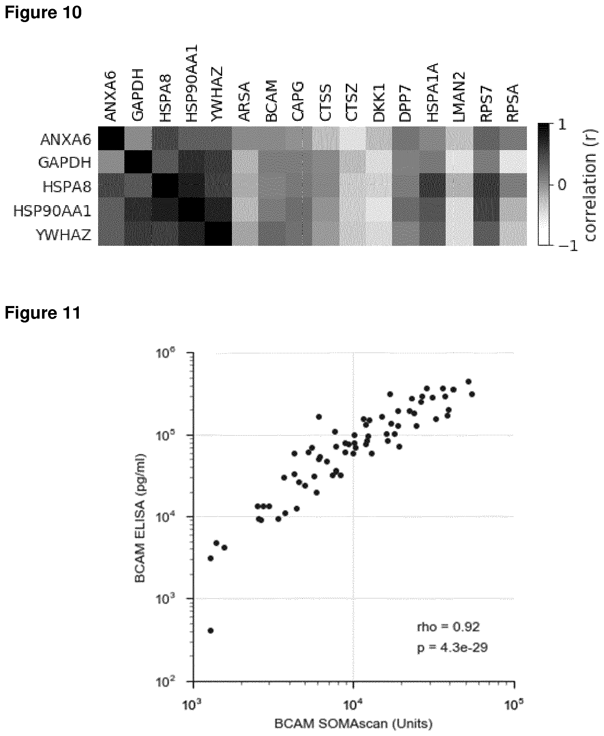

Finally, the inventors sought to shed light on the possible origin of the intracellular and membrane markers in Table 1. Toward this goal, they analyzed the correlation of these 23 markers with a panel of EV markers as well as mitochondrial cytochrome c (CYCS) as a negative EV marker indicative of apoptotic cell death. The heatmap in Figure 10 clearly suggests that several markers are likely to be associated with EVs in ascites, including BCAM, CAPG, DPP7, HAPA1A, and RPS7. This is consistent with previous proteomic studies, which identified most of the intracellular and membrane markers detected in peritoneal fluid as constituents of blood plasma or MVs in conditioned medium from tumour cells. It is therefore likely that marker as constituents of EVs play a major role in determining the survival-associated secretome of the tumour microenvironment. Therefore, the conditions of any diagnostic, prognostic or predictive assay should allow for the detection of such markers, e.g., by including detergents disrupting EVs. The standard conditions under which the SOMAscan® Proteomic Assay operates with plasma or serum include 0.05% Tween-20, which presumably allowed for the detection of MV-associated markers in the analyses. However, higher concentrations of detergents could be useful to improve the detectability of such markers.

### 5. BCAM as marker for the prognosis in OC

One of the markers with the strongest association with a poor clinical outcome is BCAM, a cell adhesion molecule functioning as a laminin receptor. This conclusion is based on the low logrank p-value determined for BCAM (Table 1) and its prevalence in prognostic signatures (Table 2). Importantly, the inventors were also able to verify the SOMAscan® Proteomic Assay results for BCAM by ELISA (rho=0.92; Figure 11). These data underscore the previously unknown clinical relevance of BCAM. Consistent with this conclusion OC is the cancer entity with the largest level of BCAM RNA and protein expression.

### 6. Methods for determining the concentration of markers

The concentration of the markers in a biological sample such as peritoneal fluid obtained from a subject with diagnosed OC are measured preferably by the aptamer-based SOMAscan® Proteomic Assay. Alternatively, the concentration of these markers can be measured by using other methods like ELISA, other antibody-based techniques, microarray-based technology, other target-proteomics-based methods or mass spectrometry.

### Description of the Figures

**Figure 1** shows the principal component analysis of peritoneal fluid (ascites; n=70), OC-plasma (n=20) and N-plasma (n=10) samples.
**Figure 2** shows the hierarchical clustering of peritoneal fluid (ascites) samples. The left cluster is cluster 1, the right cluster is cluster 2. This analysis identified two clearly separable clusters (cluster 1 and 2).
**Figure 3** shows the violin plot analysing the ratio of marker concentrations in cluster 1 versus cluster 2. Hierarchical clustering (see Figure 2) identified this ratio. p-values were determined by unpaired two-sided t-test. FC = fold change. The figure shows that the levels of 373 markers were significantly higher in cluster 1 versus cluster 2, while 406 markers showed the opposite pattern.
**Figure 4** shows the distribution of markers (proteins) associated with a positive or negative hazard ratio (HR) in clusters 1 and 2. The figure shows the vast majority of markers in cluster 1 are associated with a short relapse-free survival (RFS), whereas most markers in cluster 2 are associated with a favourable (long relapse-free) RFS.
**Figure 5** shows the functional annotations of markers filtered for cytokines and growth factors in cluster 1 (left; n=68) or cluster 2 (right; n=58) by PANTHER gene ontology (GO) enrichment analysis. In case of obvious redundancies in the search results only the term with the highest enrichment and significance was included. The figure shows that the markers in cluster 1 are predominantly associated with metastasis-associated biological processes, whereas the markers in cluster 2 are mainly linked to immune regulation.
**Figure 6** shows Kaplan-Meier plots showing examples of marker concentrations significantly associated with relapse-free survival. n: number of patients included; q: best-fit quantile used for dichotomization of patients; HR: hazard ratio (positive: shorter RFS; negative: longer RFS); rfs: median RFS for both curves.
**Figure 7** shows a patient subset simulation for the top 40 markers positively (black) or inversely (gray) associated with relapse-free survival. Connected dots: logrank test p-values (best split) determined for the complete cohort of 66 patients. Other symbols represent the p values of subset simulation, where logrank p-values were calculated for five random drawings of training and validation sets generated by randomly splitting the samples in sets of 33. Results for each pair of training and validation sets are shown as identical symbols. Median p-value of these simulations are depicted by the bar graph. Asterisks indicate p values <0.05, plus symbols a percentage of >5% of training and validation sets with significant p values.
**Figure 8** shows a graphical analysis of signature combination 1 (see Table 2), indicated at the top. The score corresponds to the number of markers above the threshold in individual patients (scores for both signatures added; maximum score = 18). POMC: Corticotropin-lipotropin. Filled triangles: correctly identified patients with relapse (uncensored). Filled dots: correctly identified relapse-free patients (censored). Asterisks: prediction not possible due to inconsistent results for both signatures.
**Figure 9** shows the bootstrapping analysis of marker combination 1 (Table 2; Figure 8). The data show 500 simulations of the groups of short-term and long-term survivors. The horizontal lines indicate the median and the 95% CI interval.
**Figure 10** shows a heatmap correlating the levels of markers in the signatures in Table 2 with extracellular vesicle (EV) markers (markers on the left-hand side). Correlation: Pearson r. Markers with levels above the median (1724 Units) of the entire SOMAscan dataset were analyzed.
**Figure 11** shows the correlation of BCAM concentrations in peritoneal fluid (ascites) measured by SOMAscan® Proteomic Assay and ELISA.

## Claims

1. Method for the determination of the prognosis of an ovarian carcinoma (OC) in a biological sample from an individual with diagnosed OC, comprising the steps
(i) measuring the concentration of at least the markers HSPA1A, BCAM, and DKK1 (markers identifying all long-term relapse-free survivors) in the biological sample of said individual;
(ii) measuring the concentration of at least the markers CAPG, LCK, and TNFAIP6 (markers identifying all short-term relapse-free survivors) in the biological sample of said individual;
(iii) determining for each marker from step (i) whether the concentration of the marker is above or below the threshold that separates short-term relapse-free survivors and long-term relapse-free survivors;
(iv) determining for each marker from step (ii) whether the concentration of the marker is above or below the threshold that separates short-term relapse-free survivors and long-term relapse-free survivors;
(v) assigning a score of 1 for all markers of step (i) with a concentration below the threshold and a score of 0 for all markers of step (i) with a concentration higher than the threshold;
(vi) assigning a score of 1 for all markers of step (ii) with a concentration below the threshold and a score of 0 for all markers of step (ii) with a concentration higher than the threshold;
(vii) addition of the scores of step (v);
(viii) addition of the scores of step (vi);
(ix) division of the sum of step (vii) by the number of the markers of step (i) in order to yield a signature score 1;
(x) division of the sum of step (viii) by the number of the markers of step (ii) in order to yield a signature score 2;
(xi) determination whether both signature scores are consistent so that both are below or above 0.5;
(xii) calculating a multi-marker score in case of consistent signatures from step (ix) and (x) by addition of the scores from step (vii) and from step (viii) followed by division by the total number of markers (number of markers in signature 1 plus number of markers in signature 2);
indicating a short-term relapse-free survival in said individual if the multi-marker score of step (xii) is 0.5 or higher, and indicating a long-term relapse-free survival in said individual if the multi-marker score of step (xii) is below 0.5.

2. Method for the determination of the prognosis of an OC in a biological sample from an individual with diagnosed OC according to claim 1, **characterized in that** the biological sample is peritoneal fluid.

3. Method for the determination of the prognosis of an OC in a biological sample from an individual with diagnosed OC according to the claims 1 or 2, **characterized in that** the determining of the concentration of said markers is performed by SOMAscan® Proteomic Assay.

4. Method for the determination of the prognosis of an OC in a biological sample from an individual with diagnosed OC according to the claims 1 or 2, **characterized in that** the determining of the concentration of said markers is performed by ELISA, other antibody-based techniques, microarray-based technology, other target-proteomics-based methods or mass spectrometry.

5. Device for the determination of the prognosis of an OC in a biological sample from an individual with diagnosed OC according to claim 1, **characterized in that** the device comprises at least
- a measuring unit for measuring the concentration of at least the markers HSPA1A, BCAM, DKK1 (markers identifying all long-term relapse-free survivors) and CAPG, LCK, TNFAIP6 (markers identifying all short-term relapse-free survivors) in the biological sample of said individual whereby this unit is capable to forward the measured concentrations to a data processing unit;
- a data processing unit which receives the measured concentrations from the measuring unit and which executes the steps (iii) to (xii) of claim 1 and forwards the results to the output unit;
- an input unit for data entry which is connectable to a database for the input of at least the threshold values and which forwards data to the data processing unit;
- an output unit for data output which indicates a short-term relapse-free survival in said individual if the multi-marker score from step (xii) of claim 1 is 0.5 or higher and indicating a long-term relapse-free survival in said individual if the multi-marker score from step (xii) of claim 1 is below 0.5.

6. Computer software to execute the method according to one of claim 1 to 4.
